**Europäisches Patentamt**

**European Patent Office**

**Office européen des brevets**

(11) Publication number: **0 125 136**
**B1**

(12) **EUROPEAN PATENT SPECIFICATION**

(45) Date of publication of patent specification: **12.09.90**

(21) Application number: **84303086.7**

(22) Date of filing: **08.05.84**

(51) Int. Cl.⁵: **C 12 Q 1/00, C 12 Q 1/48, C 12 Q 1/54**

(54) **Assay systems utilising more than one enzyme.**

(30) Priority: **05.05.83 GB 8312259**
**05.05.83 GB 8312260**
**05.05.83 GB 8312265**
**06.09.83 GB 8323797**
**06.09.83 GB 8323798**
**29.02.84 GB 8405262**
**29.02.84 GB 8405263**

(43) Date of publication of application:
**14.11.84 Bulletin 84/46**

(45) Publication of the grant of the patent:
**12.09.90 Bulletin 90/37**

(84) Designated Contracting States:
**BE CH DE FR GB IT LI NL SE**

(56) References cited:
**EP-A-0 078 636**
**AC-H-g bio che**
**FE-B-s let ter**
**FR-A-2 455 279**
**US-A-4 224 125**

(73) Proprietor: **MediSense, Inc.**
**128 Sidney Street**
**Cambridge Massachusetts 02139 (US)**

(72) Inventor: **Davis, Graham**
**6 Heron Heights**
**Goldington Green Bedfordshire (GB)**
Inventor: **Hill, Hugh Allen Oliver**
**9 Clover Close**
**Oxford OX2 9JH (GB)**

(74) Representative: **Clifford, Frederick Alan et al**
**MARKS & CLERK 57/60 Lincoln's Inn Fields**
**London WC2A 3LS (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

# EP 0 125 136 B1

**Description**

This invention relates to assay systems which direct the presence of, or measure or monitor the extent of, an enzyme-catalysed reaction on a substrate. The systems of the present invention can be used to detect or measure the enzyme or the substrate, depending upon circumstances.

Our European Patent Application 82305598 (EP—A—78636) describes and claims a sensor electrode which comprises at least at an extreme surface thereof a combination of an enzyme and a mediator compound which transfers charge to the electrode when the enzyme is catalytically active. Such an electrode, when contacting the specific substrate for the enzyme and poised at a suitable potential gives a signal responsive to the presence of, or indicative of the extent of, the enzyme/substrate reaction, even in a complex mixture of substrates since the enzyme is specific to the desired substrate component.

The practical operation of such a system depends on the incorporation of the mediator compound. A number of types of such compounds are disclosed in that application, such as polyviologens, fluoranil, chloranil, etc; but the mediators with best characteristics are metallocenes.

Ferrocenes (bis-cyclopentadienyl iron and its derivatives) fall within the last above named group and have advantages over other mediators used with enzyme/substrate reactions for charge-transfer purposes.

The unique structure and properties of ferrocene ($Fecp_2$) and its derivatives have resulted in a considerable amount of theoretical and experimental studies. First synthesised in 1951, ferrocene itself was the earliest example of the now well-known metallocene compounds.

Whilst ferrocenes had been found to be of limited value in spectrophotometric assays as a result of their poor solubility in aqueous solution and low extinction coefficients, they have been found to be more suited to a bio-electrochemical system. Ferrocenes have:

(a) wide range of redox potentials accessible through substitution of the cyclopentadienyl rings, which can be functionalised;

(b) electrochemically reversible one-electron redox properties;

(c) the pH-independent redox potential and the slow autoxidation of the reduced form.

These compounds lend themselves to the formation of derivatives, e.g. by substitution of one or both cyclopentadienyl rings and/or by polymerisation. We have studied a number of derivatives of ferrocene such as those listed in the table below:

| Ferrocene derivative | E° | Solubility |
|---|---|---|
| 1,1'-dimethyl- | 100 | I,D |
| acetic acid | 124 | S |
| hydroxyethyl- | 161 | S |
| ferrocene | 165 | I,D |
| 1,1'-bis(hydroxymethyl)- | 224 | S |
| monocarboxylic acid | 275 | S |
| 1,1'-dicarboxylic acid | 385 | S |
| chloro- | 345 | I,D |
| methyl trimethylamino- | 400 | S |

S indicates water solubility;

I,D mean respectively insoluble and detergent solubilised in 3% Tween-20.

E° is in mV vs a standard calomel electrode.

The E° values of various ferrocenes in phosphate buffer at pH 7.0 given in the above table, span a range of potentials, E°=100 to 400 mV vs SCE. The trend in E° values is in agreement with that expected on the basis of substituent effects. In general electron-donating groups stabilize the positive charge and hence promote oxidation more than electron withdrawing groups.

Of the above we find 1,1-dimethylferrocene and ferrocene monocarboxylic acid to be generally preferable because of their particularly wide range of accessible enzymes.

Although the invention described in EP—A—78636 was particularly adapted to the use of glucose as the substrate and glucose oxidase or dehydrogenase as the enzyme (thereby to provide, for example, a glucose sensor of use in the diagnosis and treatment of diabetic conditions) it was of general applicability, and other enzyme/substrate pairs such as those listed in the table following:

2

| Enzyme | Substrate |
|---|---|
| Flavo-proteins | |
| Pyruvate Oxidase | Pyruvate |
| L-Amino Acid Oxidase | L-Amino Acids |
| Aldehyde Oxidase | Aldehydes |
| Xanthine Oxidase | Xanthines |
| Glucose Oxidase | Glucose |
| Glycolate Oxidase | Glycolate |
| Sarcosine Oxidase | Sarcosine |
| Lactate Oxidase | Lactate |
| Glutathione reductase | NAD(P)H |
| Lipoamide Dehydrogenase | NADPH |
| | |
| Pyrroloquinoquinoline ("PQQ") Enzymes | |
| Glucose Dehydrogenase | Glucose |
| Methanol Dehydrogenase | Methanol and other alcohols |
| Methylamine Dehydrogenase | Methylamine |
| | |
| Haem-Containing Enzymes | |
| Lactate Dehydrogenase | Lactate |
| (Yeast Cytochrome b$_2$) | |
| Horseradish Peroxidase | Hydrogen Peroxide |
| Yeast Cytochrome c | |
| Peroxidase | Hydrogen Peroxide |
| | |
| Metalloproteins | |
| Carbonmonoxide Oxidoreductase | Carbon Monoxide |
| | |
| Cuproproteins | |
| Galactose Oxidase | Galactose |

have been shown to give useful readout signals when incorporated into such systems.

Nonetheless, not all enzyme/substrates are convenient for use in this known system, e.g. because of the unavailability or instability of the enzyme or substrate, or the difficulty in transferring to a usable form the electronic changes in the enzyme during catalytic reaction.

The present invention therefore sets out to provide a modified and elaborated system of assay utilising at least one further enzyme component.

In one aspect the invention consists in a method of assay of the type in which an electrode poised at a suitable potential is contacted with a system comprising a first enzyme, a substrate which undergoes a reaction catalysed by the said enzyme, and a mediator compound which transfers charge to the electrode from the first enzyme when it is catalytically active, whereby the current flowing in the electrode is a measure of the reaction taking place; in which the mediator is a ferrocene and at least one further enzyme and associated compound is incorporated into the system, the further enzyme being productive of, or also being reactive with, the substrate so as to affect its presence or level, but not being electrochemically linked by the mediator to the electrode, whereby the consequent difference in electrode current flowing with, and in the absence of, the second enzyme and its associated compound is a measure of the extent of reaction of the further enzyme with its associated compound and thus permits the amount of one to be established if the amount of the other is known.

EP—A—127958 of even date entitled "Analytical Equipment and Sensor Electrodes therefor" describes the nature and manufacture of sensor electrodes. Such electrodes are preferred in the practice of the present invention in which preferably the electrode is provided at its surface with the first enzyme and the mediator compound to constitute a sensor electrode.

However, EP—A—125139 of even date entitled "Assay techniques utilising Specific Binding Systems" and relating to specific binding agents (antibodies, or nucleic acid sequences) and their effect on the enzymes or mediators as an assay tool, describes systems in which the electrode can be a clean carbon rod, or having only the mediator, or only the enzyme, or occasionally include a substrate. The interested reader is referred to such systems described in EP—A—125139.

There are two main subdivisions of the present invention

(i) in which the electrode is provided at its surface with the first enzyme and the mediator compound to constitute a sensor electrode and

(ii) in which the further enzyme exerts a catalytic change on a specific further substrate to yield substrate for the first enzyme.

In the first subdivision it is preferred to operate so that (a) a sensor electrode provided at its surface with the first enzyme and with the mediator compound is contacted with a substrate to give a steady current reading (b) the second enzyme and associated compound one of which is in unknown quantity are added to set up a competitive reaction and hence decrease the electrode current and (c) the rate or extent of decrease, or the extent of substrate addition necessary to compensate for the decrease in electrode current, is noted as a measure of the amount of unknown component.

In this method the further enzyme is preferably a kinase, e.g. a hexokinase, and the associated compound a phosphate-rich compound e.g. adenosine triphosphate ("ATP").

One specifically valuable form of the invention consists in a method of assay for the unkmown one of the pair of species hexokinase and ATP, used in a combination where the amount of one is known, which comprises:

(a) contacting with a solution of glucose an electrode having at its surface a glucose oxidoreductase and a ferrocene mediator compound to transfer charge from the said enzyme to the electrode when the enzyme is catalytically active, thereby to set up a steady electrode current based on the glucose level,

(b) adding to the solution the hexokinase and ATP, so as to set up with the glucose a competitive phosphorylation to which the electrode is insensitive, whereby the steady current is correspondingly reduced and,

(c) deriving a value for the unknown level from the rate of, extent of, or glucose compensation for, the reduction in current.

Another specifically valuable form of the invention consists in a method of assay for creatine kinase, which comprises:

(a) contacting with a mixed solution of hexokinase, adenosine diphosphate, creatine phosphate and glucose an electrode having at its surface a glucose oxidoreductase enzyme and a ferrocene mediator compound to transfer charge from the enzyme to the electrode when the enzyme is catalytically active, thereby to set up a steady electrode current based on the glucose level,

(b) adding to the solution a creatine kinase to be assayed, under conditions in which adenosine diphosphate (ADP) is converted to adenosine triphosphate (ATP) by reaction of the creatine phosphate and so that consequentially the glucose is reacted, in competition with the oxidoreductase enzyme reaction, to glucose-6-phosphate by the hexokinase and ATP phosphorylation to which the electrode is insensitive, whereby the steady current is correspondingly reduced, and

(c) deriving a value for the unknown creatine kinase level from the rate of, extent of, or glucose compensation for, the reduction in current.

Yet another specifically valuable form of the invention is a method of assay for creatine which comprises:

(a) contacting with a mixed solution of hexokinase, adenosine triphosphate (ATP), creatine kinase and glucose an electrode having at its surface a glucose oxidoreductase enzyme and a ferrocene mediator compound to transfer charge from the enzyme to the electrode when the enzyme is catalytically active, thereby to set up a steady electrode current depending on the available glucose level remaining after the competitive ATP/hexokinase phosphorylation reaction to glucose-6-phosphate to which the electrode is insensitive, whereby the steady current is correspondingly reduced,

(b) adding to the solution creatine to be assayed under conditions in which ATP is converted to ADP, with the formation of creatine phosphate, by the creatine kinase, and thereby decreases the amount of ATP available for the competitive glucose phosphorylation reaction,

(c) deriving a value for the unknown creatine level from the rate of, or extent of alteration in electrode current.

The second subdivision of the invention lends itself to the fabrication of rather more elaborate electrodes. Thus, the electrode itself may be provided with the first enzyme, the mediator compound therefore, and the further enzyme to constitute a sensor electrode for the said further substrate.

For instance, the electrode may be coated with sarcosine oxidase or dehydrogenase, creatinase, and a mediator to transfer charge from the sarcosine to the electrode, and is contacted with a solution containing creatine for assay thereof by conversion to sarcosine.

For further example, the electrode may be coated with sarcosine oxidase or dehydrogenase, creatinase, creatininase, and a mediator to transfer charge from the sarcosine to the electrode, and is contacted with creatinine for assay thereof by serial conversion to creatine and sarcosine.

The preferred enzyme is a glucose oxidase or pyrroloquinoquinoline glucose dehydrogenase (for the kinase/ATP methods). One further possible addition to which this invention lends itself to the idea of chemical combination of enzyme and mediator. It is possible, as described in detail in our copending Application EP 126139 referred to above and entitled "Assay techniques utilising Specific Binding Systems", with the protein structure of a glucose oxidase, to combine chemically up to 8 to 12 ferrocene units with the enzyme without prejudicing its enzymatic activity. Such modified enzyme/mediator combinations can be used in the present invention.

The invention will be further described with reference to the accompanying drawings, in which:—

Figure 1 shows a scheme of competitive glucose oxidase/glucose and hexokinase/glucose reactions, used to detect or measure ATP or hexokinase;

Figure 2 shows a similar reaction further modified to detect or measure creatine kinase;

Figure 3 shows a similar reaction further modified to detect or measure creatine;

Figure 4 shows a scheme of reaction to detect or assay creatine;

4

Figure 5 shows graphically the effect of adding creatine kinase to a steady state solution as described with reference to Figure 12; and

Figures 6 to 13 show graphically voltammograms and derived graphical results from further specific experiments carried out.

A glucose sensor electrode was made up starting from a carbon rod. On this was deposited a solution of 1,1-dimethylferrocene in toluene. The toluene was permitted to evaporate, to leave a layer of the 1,1'-dimethylferrocene, over which was applied by carbodiimide bonding the enzyme glucose oxidase. Adjacent to but not contiguous with this composite electrode was provided a standard calomel electrode.

EP—A—78636, referred to above, and EP—A—1279258 entitled "Analytical Equipment and Sensor Electrodes therefor" describe in detail the fabrication and constructional features of numerous types of electrode of the above general type. The interested reader is referred to these patent specifications. Such a ferrocene/glucose oxidase electrode per se is not the subject of the present invention.

If the electrode as made up above is poised at +150 mV against the standard calomel electrode (SCE) it will respond, linearly over a useful range, to the available glucose level in a liquid substrate with which it is contacted.

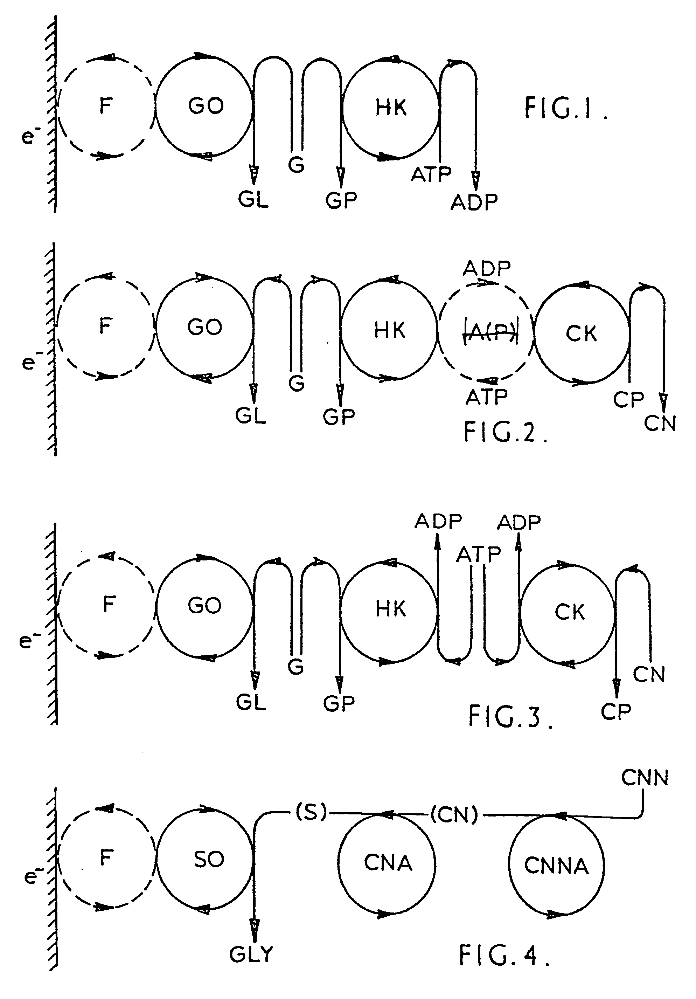

In EP—A—78636, the glucose level of a substrate was determined. In the present invention as exemplified by Figure 1, a known initial level of glucose (G) is usually used (or at least an adequate level), and a competitive reaction is set up for this glucose which can either react with the glucose oxidase (GO) to provide electron transfer, i.e. charge transfer via the ferrocene to the electrode (as before) or react with added hexokinase enzyme (HK) and adenosine triphosphate (ATP), a reaction which is not linked with the electrode. In the first case, glucose oxidation products are produced, such as gluconolactone (GL); in the second, glucose-6-phosphate (GP), with the ATP being converted to ADP (adenosine diphosphate). The higher the concentrations of ATP and HK, the greater the extent to which this separating reaction decreases the expected reading of the electrode current. If the HK concentration is known, the ATP concentration can be deduced, and vice versa. Of course, the electrode could also be used for a qualitative indication, i.e. mere detection of the HK/ATP presence.

Figure 2 illustrates another possible configuration of the assay system using an electrode made up as before. In this embodiment the assay measures the concentration of the enzyme creatine kinase (CK) (E.C. 2.7.3.2) which converts the phosphoguanidine, creatine phosphate (CP), to creatine (CN) (The system could equally well assay enzymes which have as substrates phosphoguanidines other than creatine phosphate).

In operation, a liquid system is buffered in Tris/HCl contains 20 mM Glucose (G) at pH 7, creatine phosphate (CP) (20 mM), adenosine diphosphate (ADP) (5 mM) and hexokinase (HK) 20 U/ml). It is contacted with the 1,1'-dimethylferrocene/glucose oxidase electrode, and gives a steady reading as glucose is oxidised to its oxidation product gluconolactone (GL). If creatine kinase is added then phosphate is removed from the creatine phosphate to leave creatine. This phosphate is transferred to the adenosine diphosphate (ADP) by the creatine kinase to yield adenosine triphosphate (ATP). This in turn reacts with glucose in a reaction catalysed by the enzyme hexokinase thereby phosphorylating the glucose and forming glucose-6-phosphate (GP), this reaction being competitive with the glucose oxidation reaction induced at the electrode. As a result, the concentration of the glucose in solution, and hence the current at the electrode, decreases. The phosphorylated glucose is not a substrate for the enzyme at the electrode.

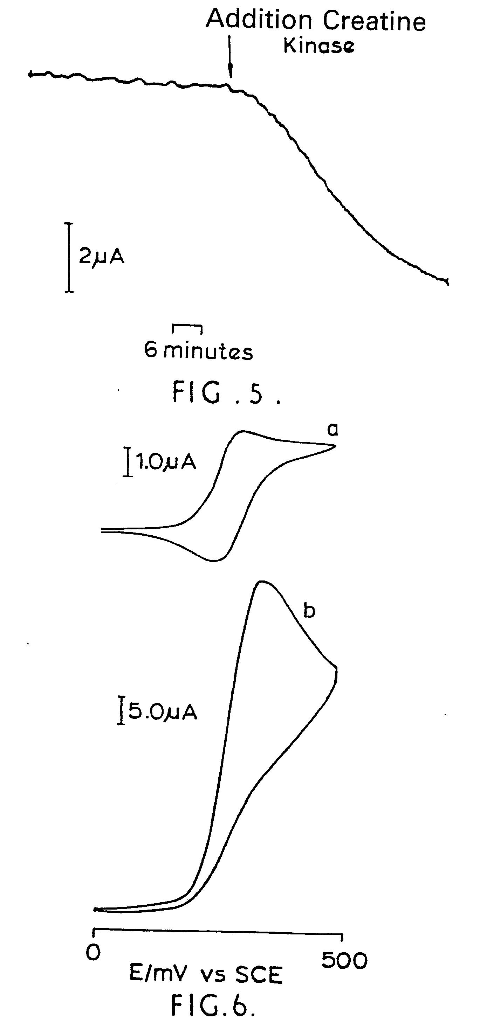

The concentration of the creatine kinase may be estimated by calculating the rate of decrease of current at the electrode; by titrating with glucose until the original current is restored; or by allowing the system to reach a steady state and comparing a final current reading with an initial current reading. Figure 5 illustrates how the initial steady catalytic current obtained with a system such as that described above with reference to Figure 2 falls when a solution containing creatine kinase is added to the system.

The creatine kinase assay give a linear response in the range $10—10^4$ Units/litre and can therefore be used in the diagnosis of a wide range of conditions, for example myocardial injuries (such as acute myocardial infarction or facultative myocardial injury) delirium tremens and muscular dystrophy.

Figure 3 illustrates a further possible embodiment which may be employed to detect the substrate creatine. In this embodiment which is buffered at pH 9, the direction of the reaction catalysed by creatine kinase is reversed.

In operation, a steady-state system is prepared as before, but with glucose (G), hexokinase (HK), creatine kinase (CK) and adenosine triphosphate (ATP). When a glucose oxidase/1,1'-dimethylferrocene electrode as described above is introduced into the solution, the steady-state electrode current will be less than that available with the glucose level above, because the adenosine triphosphate drives phosphorylation of the glucose in solution to glucose-6-phosphate (GP) and therefore reduces the activity of glucose at the electrode. In other words, a competitive reaction of known extent is set up, and stabilises.

If into this known competitive reaction a sample containing creatine is introduced the creatine will also be phosphorylated by the creatine kinase, with a corresponding reduction in the level of adenosine triphosphate; in other words a second, unknown, level of competitive reaction arises. The amount of creatine kinase available to transfer phosphate to glucose to yield glucose-6-phosphate is therefore decreased, since some is used to convert creatine to creatine phosphate.

It is possible to assay the level of creatine added if the other variables are known, or if the creatine added is known it is possible to assay the level of adenosine triphosphate, in each case simply by observing the change in activity at the electrode. An assay system according to the present invention may be used with plasma and generally speaking can be embodied for use with any biological fluid such as serum, urine, interstitial fluid, whole blood, saliva, etc. We have discovered that activity of (for example) creatine kinase in buffer, correlates well (cc≈0.99) with the activity of the enzyme in plasma.

Various modifications may be made within the scope of this embodiment. For example, a further enzyme may be incorporated to convert creatinine to creatine thus allowing assay systems to be constructed for creatinine, although Fig. 4 below shows an alternative assay system for creatinine (CNN).

Figure 4 shows a different type of assay technique which does not depend on competitive reaction for substrate, and which utilises different enzymes suitable for the assay of glycine derivatives.

In the operation of the method shown in Fig. 4 an electrode is made up as described above; but instead of immobilising glucose oxidase on the electrode surface there is immobilised on the surface a mixture of sarcosine oxidase (SO), creatininase (creatinine amidohydrolase) (CNNA), and creatinase (creatine amidinohydrolase) (CNA).

Sarcosine (N-methylgylcine) is an intermediate in the metabolism of one-carbon compounds, and a consistuent of the antinomycins, a potent group of antimetabolites.

Sarcosine oxidase is found in liver and kidney mitochondria, and may be obtained also from microbial sources for example, from cells of species of Coryneform bacteria. Sarcosine dehydrogenase may be obtained from cells of a Pseudomonad. When the electrode is contacted with a substrate containing creatinine.

(a) creatine is formed from creatinine by the action of creatininase

(b) sarcosine is formed from creatine by the enzyme creatinase and

(c) glycine is formed from sarcosine by the enzyme sarcosine oxidase, which passes electrons to the electrode via the ferrocene to give a readout signal quantitatively related to the creatinine concentration.

The electrode current measured in a suitably calibrated device is a measure of the concentration of the creatinine.

The invention will be further described with reference to the following specific Examples.

## Example 1

To the working compartment of a two-compartment electrochemical cell incorporating a 4 mm diameter gold working electrode, and 0,5 $cm^2$ platinum gauze counter electrode and a saturated calomel reference electrode. (SCE), separated from the working compartment by a Luggin capillary, was added 1 ml of Tris/HCl buffer (50 mM, pH 7.5) containing ferrocene monocarboxylic acid (200 μM), and creatinine.

Figure 6(a) shows a D.C. cyclic voltammogram obtained at a scan rate of 5 $mVs^{-1}$ over the range) to +500 mV vs SCE. The forward and reverse electrochemical waves are consistent with the reversible couple $E_{1/2}$ (Ferrocene monocarboxylic acid/ferricinium monocarboxylic acid)=+275 mV vs. SCE, and $E_{1/2}$ is the half wave potential of the system.

Subsequent serial addition of sarcosine dehydrogenase (50 IU $ml^{-1}$) and creatinine amidohydrolase (50 IU $ml^{-1}$) had no effect upon the reversible electrochemistry of ferrocenemonocarboxylic acid.

However, upon further addition of creatinase (5 mM), voltammogram (b) was obtained. The enhanced anodic current is indicative of a catalytically coupled reaction in which ferrocene monocarboxylic acid is regenerated from the ferricinium ion by reaction via reduced sarcosine hydrogenase.

This behaviour is only observed when all of the reaction components are present. Addition of creatinine, creatine and sarcosine, 5 mM respectively, to the system in the absence of any of the enzymes has no effect upon the electrochemistry of ferrocene monocarboxylic acid. Also, in another example, addition of the buffer, creatinine, ferrocene monocarboxylic acid, creatine amidinohydrolase and sarcosine dehydrogenase gave no catalytic current but only the forward and return waves for the ferrocene monocarboxylic acid until creatininase was added.

All materials that were used were supplied by Sigma Chemical Company.

## Example 2—Assay for Creatine Kinase

(a) General

Several methods for measuring CK activity in plasma have been devised and are in daily use in clinical boichemistry laboratories, to perform over 30 million determinations per year.

Creatine kinase catalyses the reversible transfer of a phosphate residue from adenosine-5'-triphosphate (ATP) to creatine.

The reaction product, creatine phosphate, represents an essential energy store for contraction, relaxation and transport of substances within muscle cells.

Creatine kinase is a dimeric enzyme, molecular weight 82 000, constituted of two subunits weighing 41 000 (3). In human tissue two different types of subunits exist, designated M (muscle) and B (brain). The dimeric enzyme can have the following forms: CM-MM skeletal muscle type, CK-BB brain type and CK-MB myocardial type. These isoenzymes can be separated by electrophoretic techniques or by an immuno-inhibition method based on the use of goat anti-human CK-MB antibody. Methods of separating the different isoenzymes are of clinical importance, since elevated isoenzyme levels in plasma have been detected and found to be associated with causes other than acute myocardial infarction (AMI) e.g. surgery, muscular dystropy and muscular injury, and strenuous exercise. Whilst this may appear to negate the significance of a total plasma CK assay to AMI, in practice the assay remains of great value since other clinical factors are also taken into account before a diagnosis is made.

The rapid response time of the ferrocene-based glucose enzyme electrodes indicates that in addition to monitoring bulk glucose concentrations, the device could be used to monitor the rates of change in bulk glucose concentrations. Thus CK activity could be determined using the coupled reaction sequence shown e.g. in Fig. 2 with the glucose enzyme electrode monitoring the rate of consumption of glucose. Under optimised conditions the rate of decrease in the electrode current should be proportional to the rate of consumption of glucose, which in turn would be proportional to the rate of consumption of creatine phosphate, from which the activity of CK can be estimated.

(b) Reagents

Creatinine phosphate, adenosine-5'-diphosphate, adenosine-5'-triphosphate, creatine kinase from rabbit muscle with an activity of 800 IU mg$^{-1}$ at 37°C and hexokinase from yeast with an activity of 1600 IU ml$^{-1}$ at 37°C were supplied by Boehringer. D-glucose and magnesium chloride were of AnalaR guide and supplied by BDH. In all experiments 25 mM tris(hydroxymethyl)aminomethane, adjusted to pH 7.0 with HCl, was used.

(c) Electrochemical experiments

D.C. cyclic voltammetry experiments were carried out with the cell described in Example 1 using a 4 mm diameter pyrolytic graphite working electrode.

Experiments in which the glucose enzyme electrode was used were performed with the first prototype design, a 1 ml three-compartment electrochemical cell, equipped with a stirrer bar. The rate of change in the steady-state glucose-dependent current was measured with the glucose enzyme electrode poised at 160 mV vs SCE, using an Oxford electrodes potentiostat and a Bryan BS-271 chart recorder.

All assays were performed under thermostatic control at 37°C.

(d) Plasma samples

Heparinised plasma samples were supplied frozen by the Clinical Biochemistry Laboratory of the John Radcliffe Hospital, Oxford.

(e) Uncoupling the glucose oxidase reaction

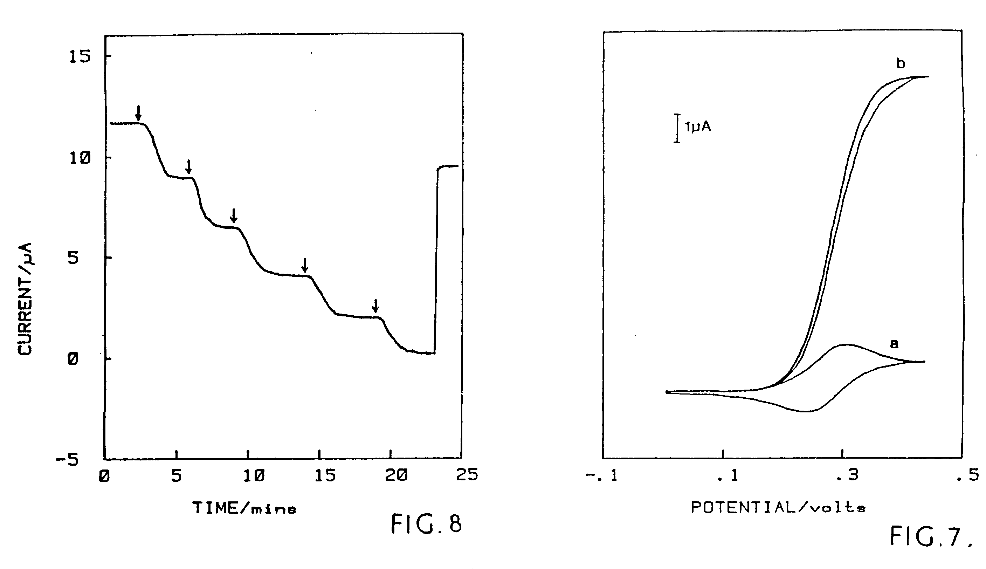

Figure 7 shows at (a) a D.C. cyclic voltammogram of ferrocene monocarboxylic acid in 25 mM Tris-HCl buffer, pH 7.0, containing 20 mM magnesium chloride and 10 mM glucose. Addition of glucose oxidase, gave curve (b) showing a typical catalytic current at oxidising potentials, resulting from the enzymatically coupled oxidation of glucose. When hexokinase 20 IU ml$^{-1}$, is added, no change in the voltammogram is observed. However, upon addition of ATP to a final concentration of 10 mM, the catalytic behaviour is no longer observed and the voltammogram associated with reversible electrochemistry of the ferrocene is again obtained, as in (a). These observations are consistent with phosphorylation of glucose to form glucose-6-phosphate, thus removing from solution the substrate for the electrochemically coupled oxidation reaction.

Since none of the components of the system interfered with the electrochemistry of ferrocene monocarboxylic acid, or showed any direct electrochemistry over the range of potential scanned, 0—450 mV vs SCE, it was possible to investigate the response of the glucose enzyme electrode, firstly as an ATP monitor, and then for monitoring kinase activity.

(f) Glucose enzyme electrode as an ATP sensor

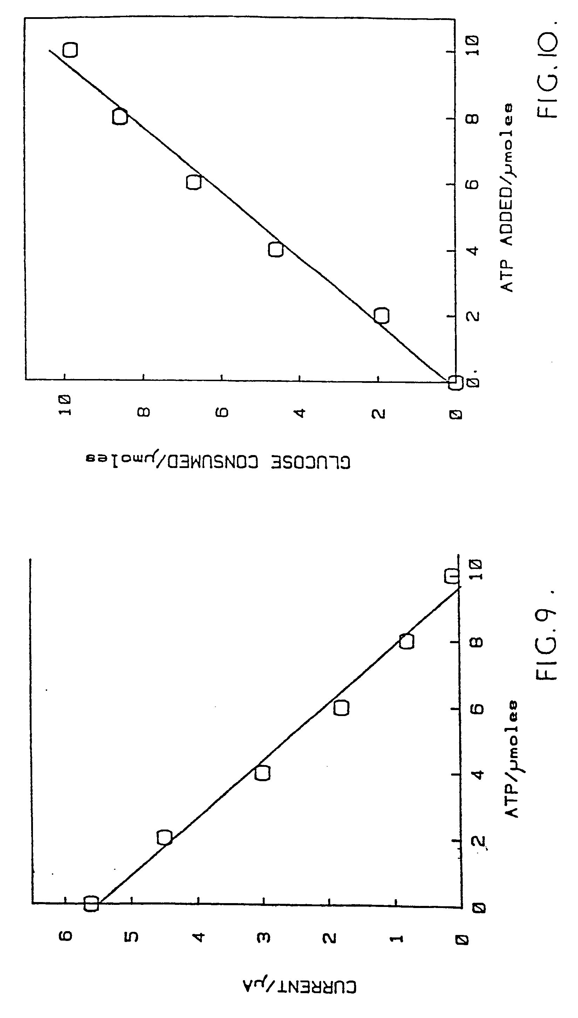

Figure 8 shows a trace of the current-time response of a glucose enzyme electrode in 10 mM glucose and hexokinase 20 IU ml$^{-1}$. A series of five aliquots of ATP (each to a final concentration of 2.0 mM) were added and the decrease in the steady-state current was measured after each addition. Figure 9 plots the steady-state current as a function of ATP added. Figure 10 correlates the amount of glucose consumed with the amount of ATP added, from which a correlation coefficient of 0.99 was calculated. This experiment show that the glucose enzyme electrode can be used to monitor stoichiometric consumption of glucose by ATP, in the presence of hexokinase. The electrode is reusable: figure 8 shows that the electrode respond to further addition of glucose at the end of the experiment.

(g) Glucose enzyme electrode based creatine kinase assay

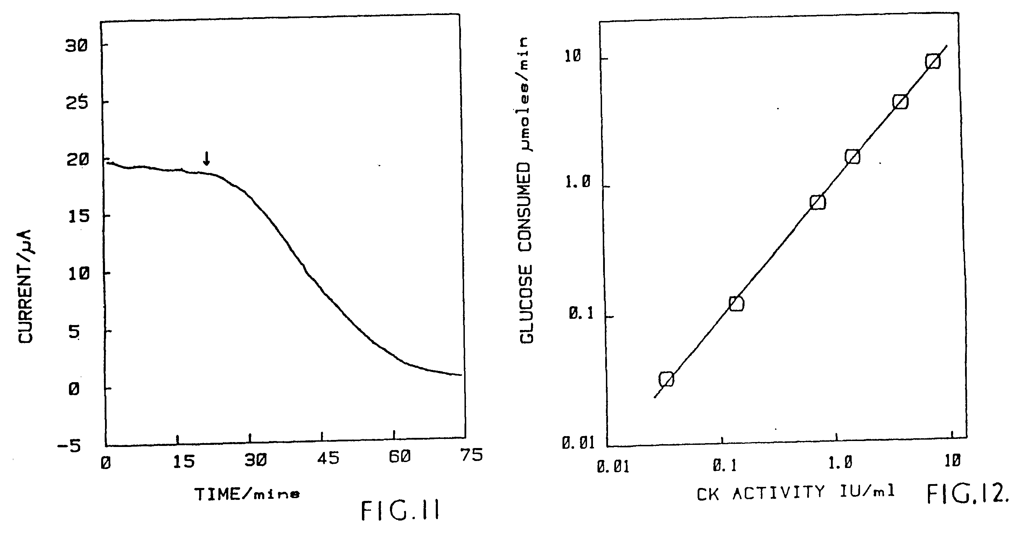

Figure 11 shows that the current-time response of the glucose enzyme electrode in 20 mM glucose is stable, after the further addition of hexokinase 20 IU ml$^{-1}$, ADP (5.0 mM) and creatine phosphate (20 mM). These reagent concentrations were found to be in sufficient excess to ensure that the rate of glucose consumption in the assay was limited by the activity of creatine kinase. When creatine kinase, with a reported activity of 500 IU l$^{-1}$ is added to the system, the current decreases with time. From the initial rate of decrease, the rate of glucose consumption was calculated. The rate of glucose consumption should be equivalent to the activity of creatine kinase in µmoles creatine phosphate consumed min$^{-1}$ mg$^{-1}$. From the results a correlation coefficient of +0.99 was calculated. (Fig. 12).

EP 0 125 136 B1

(h) Detection limits of creatine kinase assay

In post-AMI patients, plasma CK activities in the range 20—2000 IU $l^{-1}$, are common. The assay procedure, when investigated in buffered solutions, is accordingly expected to be suitable for monitoring clinical levels of CK (see Fig. 12). The upper limit of the electrode response is ca. 10 IU $ml^{-1}$ (10 000 IU $l^{-1}$).

(i) Assay of creatine kinase in plasma samples

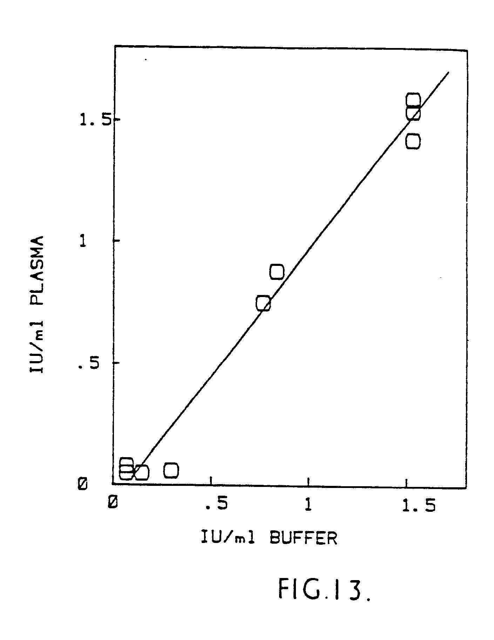

It was not possible to obtain authentic plasma samples from post-AMI patients, so samples were simulated by adding clinically relevant concentrations of CK to human plasma. Aliquots of ADP, $MgCl_2$ and HK were also added to the plasma to give the same reagent concentrations as used above. The initial glucose concentration present in the plasma was determined with the glucose enzyme electrode, and was increased to 20 mM by adding glucose. The reaction was initiated by the addition of creatine phosphate. Table II shows data obtained for the activity of creatine kinase as supplied, in buffer at pH 7.0 and in plasma at pH 7.7. The data for plasma are normalised to pH 7.0 as the activity of CK (for the reverse reaction) is lower at pH 7.7 than at pH 7.0. A correlation plot for the normalised data is presented in figure 13 from which a correlation coefficient of +0.97 was obtained.

The performance of the glucose enzyme electrode-based assay for creatine kinase in plasma suggests that the method could undergo a more detailed comparison with present methods using authentic post-AMI samples. In addition, the enzyme electrode could be further refined by the co-immobilisation of glucose oxidase and hexokinase, thus eliminating the necessity to add the latter to the assay sample.

TABLE II

| CK added IU/L | Buffer pH 7.0 IU/L | Plasma pH 7.7 IU/L | Plasma corrected to pH 7.0 IU/L |
|---|---|---|---|
| 150 | 152 | 64.7 | 154 |
| 150 | 152 | 66.8 | 159 |
| 150 | 148 | 59.6 | 142 |
| 90 | 83 | 37.0 | 88 |
| 70 | 76 | 31.5 | 75 |
| 30 | 31 | 2.5 | 5.9 |
| 15 | 15 | 1.9 | 4.5 |
| 7.5 | 7.3 | 2.3 | 5.3 |
| 7.5 | 7.3 | 3.6 | 8.6 |

**Claims**

1. A method of assay of the type in which an electrode poised at a suitable potential is contacted with a system comprising a first enzyme, a substrate which undergoes a reaction catalysed by the said enzyme, and a mediator compound which transfers charge to the electrode from the first enzyme when it is catalytically active, whereby the current flowing in the electrode is a measure of the reaction taking place: in which the mediator is a ferrocene and at least one further enzyme and associated compound is incoporated into the system, the further enzyme being productive of, or also being reactive with, the substrate so as to affect its presence or level, but not being electrochemically linked by the mediator to the electrode, whereby the consequent difference in electrode current flowing with, and in the absence of, the second enzyme and its associated compound is a measure of the extent of reaction of the further enzyme with its associated compound and thus permits the amount of one to be established if the amount of the other is known.

2. A method as claimed in Claim 1 in which the electrode is provided at its surface with the first enzyme and the mediator compound to constitute a sensor electrode.

3. A method as claimed in Claim 1 in which the further enzyme exerts a competitive reaction on the substrate and thus leads to a decrease in current flowing in the electrode.

4. A method as claimed in Claim 3 in which (a) a sensor electrode provided at its surface with the first enzyme and with the mediator compound is contacted with a substrate to give a steady current reading (b) the second enzyme and associated compound one of which is in unknown quantity are added to set up a competitive reaction and hence decrease the electrode current and (c) the rate or extent of decrease, or the extent of substrate addition necessary to compensate for the decrease in electrode current, is noted as a measure of the amount of known component.

5. A method as claimed in Claim 3 or 4 in which the further enzyme is a kinase and the associated compound is a high energy phosphate.

6. A method as claimed in Claim 3 or 4, in whihc the further enzyme is a hexokinase and the associated compound is ATP.

7. A method of assay for the unknown one of the pair of species hexokinase and ATP, used in a combination where the amount of one is known, which comprises:

(a) contacting with a solution of glucose an electrode having at its surface a glucose oxidoreductase and a mediator compound to transfer charge from the said enzyme to the electrode when the enzyme is catalytically active, thereby to set up a steady electrode current based on the glucose level;

(b) adding to the solution the hexokinase and ATP so as to set up with the glucose a competitive phosphorylation reaction to which the electrode is insensitive, whereby the steady current is correspondingly reduced;

(c) deriving a value for the unknown level from the rate of, extent of, or glucose compensation for, the reduction in current.

8. A method as claimed in Claim 1, 2, 3, 4 or 7 in which the firsm enzyme is a glucose oxidase.

9. A method as claimed in Claim 1, 2, 3, 4 or 7 in which the first enzyme is a glucose dehydrogenase.

10. A method as claimed in any preceding Claim, in which the mediator is 1,1-dimethylferrocene.

11. A sensor electrode provided at its surface with any enzyme, a ferrocene mediator compound for the enzyme, and a further enzyme whose catalytic action yields substrate for the mediated enzyme.

12. A sensor electrode according to Claim 11, wherein the mediated enzyme is chemically combined with the ferrocene mediator compound.

**Patentansprüche**

1. Ein Testverfahren der Art, bei der eine auf einem entsprechenden Potential ausgeglichene Elektrode mit einem System in Kontakt gebracht wird, das ein erstes Enzyme, ein in durch dieses Enzym katalytisch bewirkter Reaktion befindliches Substrat und eine sogenannte Beschleunigerverbindung umfaßt, die eine Ladung von dem ersten Enzym, sobald es katalytisch aktiv ist, auf die Elektrode überträgt—wobei der in der Elektrode fließende Strom ein Maß für die stattfindende Reaktion ist—, und bei der ferner der Beschleuniger ein Ferrozen ist und bei der mindestens ein weiteres Enzym und eine assoziierte Verbindung in das System eingearbeitet sind, wobei dieses weitere Enzym das Substrat produziert oder auch mit ihm reagiert, damit seine Anwesenheit oder sein Spiegel beeinflußt wird, aber wobei es nicht elektrochemisch von dem Beschleuniger an die Elektrode gebunden wird, wodurch die resultierende Differenz des mit und in Abwesenheit des zweiten Enzyms und seiner assoziierten Verbindung fließenden Elektrodenstroms ein Maß dafür ist, in welchem Umfang dieses weitere Enzyme mit seiner assoziierten Verbindung reagiert, und somit die Ermittlung der Menge des einen ermöglicht, wenn die Menge des anderen bekannt ist.

2. Ein Verfahren wie in Anspruch 1 beansprucht, bei dem das erste Enzyme und der Beschleuniger auf die Oberfläche der Elektrode aufgebracht werden, um eine Sensorelekrode zu bilden.

3. Ein Verfahren wie in Anspruch 1 beansprucht, bei dem das weitere Enzyme eine Parallelreaktion bei dem Substrat bewirkt und dadurch zu einer Reduzierung des in der Elektrode fließenden Stroms führt.

4. Ein Verfahren wie in Anspruch 3 beansprucht, bei dem (a) eine auf der Oberfläche mit dem ersten Enzym und dem Beschleuniger versehene Sensorelektrode mit einem Substrat in Kontakt gebracht wird, damit der Strom kontinuierlich gemessen werden kann, bei dem (b) das zweite Enzym und die assoziierte Verbindung—eine dieser Substanzen in unbekannter Menge—hinzugegeben werden, um eine Parallelreaktion einzuleiten und dadurch den Elektrodenstrom zu reduzieren, und bei dem (c) Geschwindigkeit oder Ausmaß der Stromabnahme oder aber die Menge der zum Ausgleich der Elektrodenstromabnahme notwendigen Substratzugabe als Maß für die Menge eines unbekannten Bestandteils erfaßt wird.

5. Ein Verfahren wie in Anspruch 3 oder 4 beansprucht, bei dem das weitere Enzym ein Kinase und die assoziierte Verbindung ein energiereiches Phosphat sind.

6. Ein Verfahren wie in Anspruch 3 oder 4 beansprucht, bei dem das weitere Enzym eine Hexokinase und die assoziierte Verbindung ATP sind.

7. Ein Testverfahren zur Ermittlung der einen unbekannten Substanz des Paars Gattung Hexokinase/ATP, das in einer Kombination verwendet wird, bei der die Menge der einen Substanz bekannt ist; dieses Testverfahren umfaßt folgendes:

a) Eine Elektrode—auf deren Oberfläche Glukose-Oxidoreduktase und eine Beschleunigerverbindung zur Ableitung der Ladung von dem genannten Enzym auf die Elektrode, sobald das Enzym katalytisch aktiv ist, aufgebracht werden, um dadurch einen Dauerelektrodenstrom auf der Basis des Glukosespiegels zu erzeugen—wird mit einer Glukoselösung in Kontakt gebracht;

b) der Lösung werden die Hexokinase und das ATP zugegeben, um damit eine parallel und die Elektrode nicht beeinflussende Phosphorylierungsreaktion mit der Glukose in Gang zu setzen, wodurch der Dauerstrom entsprechend reduziert wird;

c) aus der Geschwindigkeit oder der Größe der Stromabnahme oder aus der zur Kompensierung der Stromabnahme notwendigen Glukose wird ein Wert für die unbekannte Menge abgeleitet.

8. Ein Verfahren wie in Anspruch 1, 2, 3, 4 oder 7 beansprucht, bei dem das erste Enzym eine Glukose-Oxidase ist.

9. Ein Verfahren wie in Anspruch 1, 2, 3, 4 oder 7 beansprucht, bei dem das erste Enzym eine Glukose-Dehydrogenase ist.

10. Ein Verfahren wie in einem der vorstehenden Ansprüche beansprucht, bei dem der Beschleuniger 1,1-Dimethylferrozen ist.

11. Eine Sensorelektrode, die auf ihrer Oberfläche mit irgendeinem Enzym, einer Ferrozen-

# EP 0 125 136 B1

Beschleunigerverbindung für das Enzym und einem weiteren Enzym versehen ist, dessen katalytische Reaktion ein Substrat für das reaktionsbeschleunigte Enzym ergibt.

12. Eine Sensorelektrode nach Anspruch 11, bei dem das reaktionsbeschleunigte Enzym chemisch mit der Ferrozen-Beschleunigerverbindung kombiniert wird.

## Revendications

1. Procédé d'essai (de détection ou de dosage), du type dans lequel une électrode, réglée à un potentiel convenable, est mise en contact avec un système comprenant une première enzyme, un substrat qui subit une réaction catalysée par ladite enzyme, et un composé médiateur, qui transfère à l'électrode une charge provenant de la première enzyme quand elle est catalytiquement active, de sorte que le courant circulant dans l'électrode constitue une mesure de la réaction qui se produit; procédé dans lequel le médiateur est un ferrocène et au moins une enzyme supplémentaire et un composé associé sont incorporés au système, l'enzyme supplémentaire étant capable de produire le substrat, ou pouvant également réagir avec le substrat, de façon à influer sur la présence ou le taux de ce substrat, mais n'étant pas électrochimiquement liée par le médiateur à l'électrode, de sorte que la différence, qui en résulte, entre le courant circulant dans l'électrode, en cas de présence de la seconde enzyme et du composé qui lui est associé, et en cas d'absence de la seconde enzyme et du composé qui lui est associé, constitue une mesure de l'étendue ou du degré de réaction de l'enzyme supplémentaire avec le composé qui lui est associé et permet ainsi d'établir la quantité de l'un si l'on connaît la quantité de l'autre.

2. Procédé tel que revendiqué à la revendication 1, dans lequel l'électrode comporte à sa surface la première enzyme et le composé médiateur pouc constituer une électrode détectrice.

3. Procédé tel que revendiqué à la revendication 1, dans lequel l'enzyme supplémentaire effectue une réaction de compétition sur le substrat et provoque ainsi une diminution du courant passant dans l'électrode.

4. Procédé tel que revendiqué à la revendication 3, dans lequel (a) une électrode détectrice, comportant à sa surface la première enzyme et le composé médiateur, est mise en contact avec un substrat pour donner une lecture constante du courant; (b) on ajoute la seconde enzyme et le composé associé, dont l'un ou l'une est présent(e) en une quantité inconnue, pour provoquer une réaction de compétition et diminuer donc le courant passant dans l'électrode et (c) on note la vitesse et l'étendue de la diminution, ou l'étendue de l'addition de substrat nécessaire pour compenser la diminution du courant d'électrode, à titre de mesure de la quantité du constituant inconnu.

5. Procédé tel que revendiqué à la revendication 3 ou 4, dans lequel l'enzyme supplémentaire est une kinase et la composé associé est un phosphate à haute énergie.

6. Procédé tel que revendiqué à la revendication 3 ou 4, dans lequel l'enzyme supplémentaire est une hexokinase et le composé associé est ATP.

7. Procédé pour doser la partie inconnue de la paire constituée par une hexokinase et de l'ATP, que l'on utilise en combinaison, la quantité de l'une des parties étant connue, ce procédé comprenant:

(a) la mise en contact, avec une solution de glucose, d'une électrode comportant à sa surface une glucose oxydoréductase et un composé médiateur pour transférer une charge de ladite enzyme à l'électrode lorsque l'enzyme est catalytiquement active, de façon à provoquer le passage d'un courant stable d'électrode sur la base du taux de glucose,

(b) l'addition, à la solution, de l'hexokinase et de l'ATP de façon à provoquer, avec le glucose, une réaction de phosphorylation entrant en compétition et à laquelle l'électrode est insensible, de sorte que le courant constant et régulier est diminué de manière correspondante;

(c) l'obtention (par le calcul) d'une valeur du taux inconnu, que l'on obtient à partir de la vitesse de diminution du courant, de l'amplitude de cette diminution ou de la quantité de glucose ajoutée pour compenser cette diminution.

8. Procédé tel que revendiqué dans les revendications 1, 2, 3, 4 et 7, dans lequel la première enzyme est une glucose oxydase.

9. Procédé tel que revendiqué à la revendication 1, 2, 3, 4 ou 7, dans lequel la première enzyme est une glucose déshydrogénase.

10. Procédé tel que revendiqué dans l'une quelconque des revendications précédentes, dans lequel le médiateur est le 1,1'-diméthylferrocène.

11. Electrode détectrice comportant à sa surface une enzyme, un ferrocène constituant un composé médiateur pour l'enzyme, et une enzyme supplémentaire dont l'action catalytique donne un substrat pour l'enzyme associée à son médiateur.

12. Electrode détectrice selon la revendication 11, dans laquelle l'enzyme associée à son médiateur est chimiquement combinée au ferrocène constituant le composé médiateur.

FIG.1.

FIG.2.

FIG.3.

FIG.4.

EP 0 125 136 B1

Addition Creatine Kinase

2µA

6 minutes

FIG . 5 .

a

1.0µA

b

5.0µA

0    E/mV vs SCE    500

FIG.6.

FIG.8

FIG.7.

EP 0 125 136 B1

FIG.IO.

ATP ADDED/μmoles

GLUCOSE CONSUMED/μmoles

FIG.9.

ATP/μmoles

CURRENT/μA

FIG.11

FIG.12.

EP 0 125 136 B1

FIG. 13.